# EUROPEAN PATENT APPLICATION

(11) **EP 3 696 268 A1**
(43) Date of publication of application: **19.08.2020**
(21) Application number: 18866008.8
(22) Date of filing: 12.10.2018
(51) Int. Cl.: C12N 15/11, C12M 1/00, C12Q 1/686, C12Q 1/689

(54) **mecA GENE AMPLIFICATION PRIMER PAIR, mecA GENE DETECTION KIT AND mecA GENE DETECTION METHOD**

(30) Priority: 12.10.2017 JP 2017198576
(71) Applicant: Mitsui Chemicals, Inc., Minato-ku Tokyo 105-7122 (JP)
(72) Inventor: AMANO Koh, Mobara-shi Chiba 297-0017 (JP); ENDO Ayako, Mobara-shi Chiba 297-0017 (JP); YANAI Hisaaki, Mobara-shi Chiba 297-0017 (JP); TSUJI Kentaro, Mobara-shi Chiba 297-0017 (JP); MORISHIGE Takashi, Tokyo 105-7122 (JP)
(74) Representative: Mewburn Ellis LLP
(86) International application number: PCT/JP2018/038072
(87) International publication number: WO 2019/074091

(57) **Abstract**

Provided is a primer pair of primers for methicillin-resistant gene detection for the purpose of achieving highly sensitive methicillin-resistant gene detection. Said primer pair comprises a combination of SEQ ID NO: 3 and SEQ ID NO: 7, a combination of SEQ ID NO: 2 and SEQ ID NO: 9, a combination of SEQ ID NO: 1 and SEQ ID NO: 8, a combination of SEQ ID NO: 1 and SEQ ID NO: 9, a combination of SEQ ID NO: 4 and SEQ ID NO: 11, a combination of SEQ ID NO: 5 and SEQ ID NO: 12, a combination of SEQ ID NO: 6 and SEQ ID NO: 10, or a combination of SEQ ID NO: 6 and SEQ ID NO: 12.

## Description

### Technical Field

The present invention relates to primer pairs, kit and method for conveniently, quickly and sensitively detecting mecA genes, which are specific genetic elements of methicillin resistance.

### Technical Background

Methicillin resistant Staphylococcus aureus (abbreviated as "MRSA") has become the leading cause of nosocomial infections worldwide and is regarded as the most clinically relevant drug-resistant organism in many countries. mecA, a gene involved in methicillin resistance in MRSA, encodes PBP (Penicillin Binding Protein)-2' (also called PBP-2a). Usually, Staphylococcus aureus produces mainly four PBPs. PBP is a protein involved in cell wall peptidoglycan synthesis and has transpeptidase activity. β-Lactam antibiotics act by binding to the active site of PBP and inhibiting transpeptidase activity and peptidoglycan synthesis. However, PBP-2' avoids the inhibitory effects of peptidoglycan syntheses by lowering the affinity for β-lactam antibiotics (Non-Patent Document 1).
In Staphylococcus aureus and coagulase-negative staphylococci, the presence or absence of methicillin resistance is very important information in the diagnosis of infected patients and in the decision of treatment plan. Although Staphylococcus aureus is more important than other coagulase-negative staphylococci because of its greater virulence, infection with a compromised host is also a major problem for methicillin-resistant Staphylococcus aureus and coagulase-negative staphylococci. Therefore, rapid detection of methicillin resistance for rapid diagnosis and treatment has been desired as a countermeasure against nosocomial infection of methicillin -resistant staphylococci.
Methicillin resistance has been tested by methods such as drug sensitivity tests, immunoassays, and PCR (polymerase chain reaction) detections (Non-Patent Document 2).
Drug sensitivity tests require that blood from the patient be cultured for at least one day to increase staphylococci. Immunoassays also require a step of culturing staphylococci and therefore require at least one day before the results of methicillin resistance tests can be obtained.
In comparison, nucleic acid amplification tests, such as PCR, are very sensitive and do not require a culturing process and can be tested directly from the patient's blood. Therefore, the test result can be submitted several hours after the arrival of the patient blood sample (Patent Document 1).

### Prior-art document

[Patent Document 1] International Publication No. WO 2007/097323

[Non-Patent Document 1] Journal of Bacteriology, May 1980 p.275
[Non-Patent Document 2] Journal of Clinical Microbiology, July 1992, p.1685

### SUMMARY OF THE INVENTION

### Problems to be Solved

Although several methods for detecting methicillin resistance genes by nucleic acid amplification such as PCR have already been reported, there are many reports in which specific detection sensitivity has not been specified. Even if the detection sensitivity is specified, there is only a report that the detection sensitivity is not sufficient for use in a clinical field (Non-Patent Document 2).
On the other hand, detection of the methicillin resistance gene greatly influences the diagnosis and treatment plan of the patient, and therefore, development of a method for detecting the methicillin resistance gene quickly and accurately and reliably is an urgent clinical necessity. Therefore, it is an object of the present invention to quickly and accurately and reliably detect a methicillin resistance gene. It is an object of the present invention to provide a primer pair for realizing particularly sensitive detection of a methicillin resistance gene.

### Means for Solving the Problems

The present invention is a method for quickly identifying a methicillin resistance gene directly from a patient sample by using a nucleic acid amplification method, and reliably detecting the methicillin resistance gene with higher detection sensitivity than the conventional method. The present inventors have focused on PCR for the purpose of establishing a highly sensitive and rapid method for detecting methicillin resistance genes. In particular, primers used in the PCR method were examined in order to detect the methicillin resistance gene with high sensitivity.
The primer was designed under the following conditions.
The size of the DNA fragment to be amplified was increased within a range in which the amplification efficiency was not lowered. Specifically, the size of the DNA fragment was 150bp or more and 700bp or less. By increasing the sizes of the DNA fragments to be amplified, the fluorescent intensities of the amplification curves during the PCR reactions were increased, and height of the peaks obtained by Melting analysis was also increased.
The sequence of mecA homolog is confirmed, and the base sequence of the primer pair is designed only in the highly conserved part. Specifically, those containing a sequence with low conservation within 5 bases from the 3' end of the primer was excluded, and the base sequence was modified to obtain a highly conserved sequence.
Based on the above-described concepts, 32 specific Fwd and Rev primers were prepared, respectively, and 313 primer pairs considered to be promising were produced by combining them, from which optimum combinations were examined to select primer pairs capable of achieving high detection sensitivity compared with Patent Document 1, which was the prior application.
That is, the primer pair is a primer pair for detecting a mecA gene (methicillin resistance gene), and the primer pair is one primer pair selected from the group consisting of (a) to (c) below.
(a) A primer pair comprising a combination of SEQ ID NO:3 and SEQ ID NO:7, a combination of SEQ ID NO:2 and SEQ ID NO:9, a combination of SEQ ID NO:1 and SEQ ID NO:8, a combination of SEQ ID NO:1 and SEQ ID NO:9, a combination of SEQ ID NO:4 and SEQ ID NO:11, a combination of SEQ ID NO:5 and SEQ ID NO: 12, a combination of SEQ ID NO:6 and SEQ ID NO:10 or a combination of SEQ ID NO:6 and SEQ ID NO: 12,
(b) In (a) above, a primer pair in which 1 to 2 bases are added, deleted or substituted to the extent that a part of the base sequence of one or both primers does not impair the function as a primer,
(c) A primer pair comprising a complementary sequence corresponding to the base sequence of (a) or (b) above.

A mecA gene detection kit according to the present invention includes at least one primer pair selected from the group consisting of (a) to (c) above. A method for detecting mecA in a sample according to the present invention,
PCR using DNA prepared from the sample and primer pairs for amplifying the DNA,
Detection of a mecA gene in the sample by detection of a mecA gene in an amplification product obtained by the PCR-process or by analyzing the amplification product,

### With,

The primer pair used in the PCR step is at least one primer pair selected from the group consisting of (a) to (c) above.
It is characterized by:

### Effect of Invention

According to the present disclosure, primer pairs and mecA gene detection kits for PCR for highly sensitive mecA gene detection, and a detection method of a mecA gene can be provided. Therefore, according to the present embodiment, it is possible to detect a trace amount of mecA gene in a sample in a medical field, a food field, or the like with higher sensitivity and higher accuracy than conventional test kits.

### BRIEF DESCRIPTION OF THE DRAWINGS

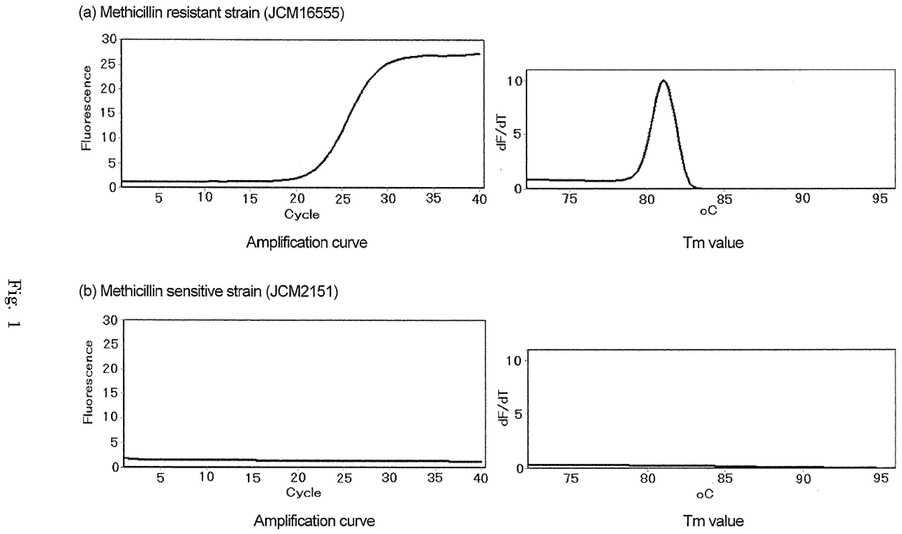

Figure 1 shows a comparison of the results of measurements on methicillin-resistant and methicillin-sensitive strains. FIG. 1(a) shows the results of measurement of amplification curves and Tm values when samples from methicillin-resistant strains are used, and FIG. 1(b) shows the results of measurement of amplification curves and Tm values when samples from methicillin-sensitive strains are used.

### DETAILED DESCRIPTION OF THE PRESENT INVENTION

### <primer>

In the present invention, a primer refers to a DNA which becomes a starting point at the time of DNA replication in the PCR method. In the present specification, unless otherwise indicated, a primer refers to a DNA primer.
The point of the present invention lies in a specific primer pair that can enjoy the effect of improving detection sensitivity.
A primer pair in Patent Document 1 consisting of a base sequence of sequence number 45 and a base sequence of sequence number 46 has DNA sequences that hybridize to the sequence in the structural gene of the methicillin-resistant gene (mecA). The base sequence of SEQ ID NO: 45 and the base sequence of SEQ ID NO: 46 described in Patent Document 1 are referred to as SEQ ID NOs: 13 and 14, respectively, in this specification. However, even in the above-mentioned primer pairs, the PCR-based detection methods did not have adequate detection sensitivities, and low concentrations of mecA could not be detected (Examples 5 and table 2 to be described later). This may indicate false negatives due to insensitivity when assaying specimens containing low concentrations of MRSA.

The structural gene of mecA gene is composed of approximately 2100bp, and it has been found that mecA gene can be detected more sensitively by performing PCR using each of the primer pairs included in the following (a) group designed by the present inventors to hybridize within the structural gene.
(a) group:
   (a1) A primer pair consisting of a combination of a primer consisting of SEQ ID NO: 3 and a primer consisting of SEQ ID NO: 7.
   (a2) A primer pair consisting of a combination of a primer consisting of SEQ ID NO: 2 and a primer consisting of SEQ ID NO: 9.
   (a3) A primer pair consisting of a combination of a primer consisting of SEQ ID NO: 1 and a primer consisting of SEQ ID NO: 8.
   (a4) A primer pair consisting of a combination of a primer consisting of SEQ ID NO: 1 and a primer consisting of SEQ ID NO: 9.
   (a5) A primer pair consisting of a combination of a primer consisting of SEQ ID NO: 4 and a primer consisting of SEQ ID NO: 11.
   (a6) A primer pair consisting of a combination of a primer consisting of SEQ ID NO: 5 and a primer consisting of SEQ ID NO: 12.
   (a7) A primer pair consisting of a combination of a primer consisting of SEQ ID NO: 6 and a primer consisting of SEQ ID NO: 10.
   (a8) A primer pair consisting of a combination of a primer consisting of SEQ ID NO: 6 and a primer consisting of SEQ ID NO: 12.

As long as the effect of the present invention can be enjoyed, a modified primer consisting of a modified base sequence obtained by converting a part of bases in the base sequence of the DNA fragment (oligonucleotide) constituting each of the above-mentioned primers can also be applied. In principle, a modified primer is considered equivalent to each primer if it is capable of hybridizing with the complementary DNA strand of each primer described above. Further, it is preferable that the modified primer considered as equivalent is a primer having a function as a primer which hybridizes to the complementary DNA strand of the original primer, and having a modified base sequence in which one or two bases are added, deleted, or substituted to the base sequence of the original primer. Therefore, primer pairs included in the following (b) group can also be used as primer pairs for detecting mecA genes.
(b) group:
(b1-1) A primer pair consisting of a combination of a modified primer consisting of the modified base sequence of SEQ ID NO: 3 and a primer consisting of SEQ ID NO: 7.
(b1-2) A primer pair consisting of a combination of a primer consisting of SEQ ID NO: 3 and a primer consisting of a modified base sequence of SEQ ID NO: 7.
(b1-3) A primer pair consisting of a combination of a modified primer consisting of the modified base sequence of SEQ ID NO: 3 and a modified primer consisting of the modified base sequence of SEQ ID NO: 7.
(b2-1) A primer pair consisting of a combination of a modified primer consisting of the modified base sequence of SEQ ID NO: 2 and a primer consisting of SEQ ID NO: 9.
(b2-2) A primer pair consisting of a combination of a primer consisting of SEQ ID NO: 2 and a primer consisting of a modified base sequence of SEQ ID NO: 9.
(b2-3) A primer pair consisting of a combination of a modified primer consisting of the modified base sequence of SEQ ID NO: 2 and a primer consisting of the modified base sequence of SEQ ID NO: 9.
(b3-1) A primer pair consisting of a combination of a modified primer consisting of the modified base sequence of SEQ ID NO: 1 and a primer consisting of the base sequence of SEQ ID NO: 8.
(b3-2) A primer pair consisting of a combination of a primer consisting of SEQ ID NO: 1 and a primer consisting of a modified base sequence of SEQ ID NO: 8.
(b3-3) A primer pair consisting of a combination of a modified primer consisting of the modified base sequence of SEQ ID NO: 1 and a modified primer consisting of the modified base sequence of SEQ ID NO: 8.
(b4-1) A primer pair consisting of a combination of a modified primer consisting of the modified base sequence of SEQ ID NO: 1 and a primer consisting of SEQ ID NO: 9.
(b4-2) A primer pair consisting of a combination of a primer consisting of SEQ ID NO: 1 and a primer consisting of a modified base sequence of SEQ ID NO: 9.
(b4-3) A primer pair consisting of a combination of a modified primer consisting of the modified base sequence of SEQ ID NO: 1 and a modified primer consisting of the modified base sequence of SEQ ID NO: 9.
(b5-1) A primer pair consisting of a combination of a modified primer consisting of the modified base sequence of SEQ ID NO: 4 and a primer consisting of SEQ ID NO: 11.
(b5-2) A primer pair consisting of a combination of a primer consisting of SEQ ID NO: 4 and a primer consisting of a modified base sequence of SEQ ID NO: 11.
(b5-3) A primer pair consisting of a combination of a modified primer consisting of the modified base sequence of SEQ ID NO: 4 and a modified primer consisting of the modified base sequence of SEQ ID NO: 11.
(b6-1) A primer pair consisting of a combination of a modified primer consisting of the modified base sequence of SEQ ID NO: 5 and a primer consisting of SEQ ID NO: 12.
(b6-2) A primer pair consisting of a combination of a primer consisting of SEQ ID NO: 5 and a primer consisting of a modified base sequence of SEQ ID NO: 12.
(b6-3) A primer pair consisting of a combination of a modified primer consisting of the modified base sequence of SEQ ID NO: 5 and a modified primer consisting of the modified base sequence of SEQ ID NO: 12.
(b7-1) A primer pair consisting of a combination of a modified primer consisting of the modified base sequence of SEQ ID NO: 6 and a primer consisting of SEQ ID NO: 10.
(b7-2) A primer pair consisting of a combination of a primer consisting of SEQ ID NO: 6 and a primer consisting of a modified base sequence of SEQ ID NO: 10.
(b7-3) A primer pair consisting of a combination of a modified primer consisting of the modified base sequence of SEQ ID NO: 6 and a modified primer consisting of the modified base sequence of SEQ ID NO: 10.
(b8-1) A primer pair consisting of a combination of a modified primer consisting of the modified base sequence of SEQ ID NO: 6 and a primer consisting of SEQ ID NO: 12.
(b8-2) A primer pair consisting of a combination of a primer consisting of SEQ ID NO: 6 and a primer consisting of a modified base sequence of SEQ ID NO: 12.
(b8-3) A primer pair consisting of a combination of a modified primer consisting of the modified base sequence of SEQ ID NO: 6 and a modified primer consisting of the modified base sequence of SEQ ID NO: 12.

Furthermore, a pair of complementary primers composed of complementary sequences of primers constituting the respective primer pairs can also be used as a primer pair for detecting mecA genes. That is, a primer pair included in the following (c) group can also be used as a primer pair for detecting mecA genes.
(c) group:
(c1-1) A primer pair consisting of a combination of a primer consisting of the complementary sequence of SEQ ID NO: 3 and a primer consisting of the complementary sequence of SEQ ID NO: 7.
(cl-2) A primer pair consisting of a combination of a modified primer consisting of the complementary sequence of the modified base sequence of SEQ ID NO: 3 and a primer consisting of the complementary sequence of SEQ ID NO: 7.
(c1-3) A primer pair consisting of a combination of a primer consisting of the complementary sequence of SEQ ID NO: 3 and a primer consisting of the complementary sequence of the modified base sequence of SEQ ID NO: 7.
(c1-4) A primer pair consisting of a combination of a modified primer consisting of the complementary sequence of the modified base sequence of SEQ ID NO: 3 and a modified primer consisting of the complementary sequence of the modified base sequence of SEQ ID NO: 7.
(c2-1) A primer pair consisting of a combination of a primer consisting of the complementary sequence of SEQ ID NO: 2 and a primer consisting of the complementary sequence of SEQ ID NO: 9.
(c2-2) A primer pair consisting of a combination of a modified primer consisting of the complementary sequence of the modified base sequence of SEQ ID NO: 2 and a primer consisting of the complementary sequence of SEQ ID NO: 9.
(c2-3) A primer pair comprising a combination of a primer consisting of the complementary sequence of SEQ ID NO: 2 and a primer consisting of the complementary sequence of the modified base sequence of SEQ ID NO: 9.
(c2-4) A primer pair consisting of a combination of a modified primer consisting of the complementary sequence of the modified base sequence of SEQ ID NO: 2 and a modified primer consisting of the complementary sequence of the modified base sequence of SEQ ID NO: 9.
(c3-1) A primer pair consisting of a combination of a primer consisting of the complementary sequence of SEQ ID NO: 1 and a primer consisting of the complementary sequence of SEQ ID NO: 8.
(c3-2) A primer pair consisting of a combination of a modified primer consisting of the complementary sequence of the modified base sequence of SEQ ID NO: 1 and a primer consisting of the complementary sequence of the base sequence of SEQ ID NO: 8.
(c3-3) A primer pair comprising a combination of a primer consisting of the complementary sequence of SEQ ID NO: 1 and a primer consisting of the complementary sequence of the modified base sequence of SEQ ID NO: 8.
(c3-4) A primer pair consisting of a combination of a modified primer consisting of the complementary sequence of the modified base sequence of SEQ ID NO: 1 and a modified primer consisting of the complementary sequence of the modified base sequence of SEQ ID NO: 8.
(c4-1) A primer pair consisting of a combination of a primer consisting of the complementary sequence of SEQ ID NO: 1 and a primer consisting of the complementary sequence of SEQ ID NO: 9.
(c4-2) A primer pair consisting of a combination of a modified primer consisting of the complementary sequence of the modified base sequence of SEQ ID NO: 1 and a primer consisting of the complementary sequence of SEQ ID NO: 9.
(c4-3) A primer pair comprising a combination of a primer consisting of the complementary sequence of SEQ ID NO: 1 and a primer consisting of the complementary sequence of the modified base sequence of SEQ ID NO: 9.
(c4-4) A primer pair consisting of a combination of a modified primer consisting of the complementary sequence of the modified base sequence of SEQ ID NO: 1 and a modified primer consisting of the complementary sequence of the modified base sequence of SEQ ID NO: 9.
(c5-1) A primer pair consisting of a combination of a primer consisting of the complementary sequence of SEQ ID NO: 4 and a primer consisting of the complementary sequence of SEQ ID NO: 11.
(c5-2) A primer pair consisting of a combination of a modified primer consisting of the complementary sequence of the modified base sequence of SEQ ID NO: 4 and a primer consisting of the complementary sequence of SEQ ID NO: 11.
(c5-3) A primer pair comprising a combination of a primer consisting of the complementary sequence of SEQ ID NO: 4 and a primer consisting of the complementary sequence of the modified base sequence of SEQ ID NO: 11.
(c5-4) A primer pair consisting of a combination of a modified primer consisting of the complementary sequence of the modified base sequence of SEQ ID NO: 4 and a modified primer consisting of the complementary sequence of the modified base sequence of SEQ ID NO: 11.
(c6-1) A primer pair consisting of a combination of a primer consisting of the complementary sequence of SEQ ID NO: 5 and a primer consisting of the complementary sequence of SEQ ID NO: 12.
(c6-2) A primer pair consisting of a combination of a modified primer consisting of the complementary sequence of the modified base sequence of SEQ ID NO: 5 and a primer consisting of the complementary sequence of SEQ ID NO: 12.
(c6-3) A primer pair comprising a combination of a primer consisting of the complementary sequence of SEQ ID NO: 5 and a primer consisting of the complementary sequence of the modified base sequence of SEQ ID NO: 12.
(c6-4) A primer pair consisting of a combination of a modified primer consisting of the complementary sequence of the modified base sequence of SEQ ID NO: 5 and a modified primer consisting of the complementary sequence of the modified base sequence of SEQ ID NO: 12.
(c7-1) A primer pair consisting of a combination of a primer consisting of the complementary sequence of SEQ ID NO: 6 and a primer consisting of the complementary sequence of SEQ ID NO: 10.
(c7-2) A primer pair consisting of a combination of a modified primer consisting of the complementary sequence of the modified base sequence of SEQ ID NO: 6 and a primer consisting of the complementary sequence of SEQ ID NO: 10.
(c7-3) A primer pair consisting of a combination of a primer consisting of the complementary sequence of SEQ ID NO: 6 and a primer consisting of the complementary sequence of the modified base sequence of SEQ ID NO: 10.
(c7-4) A primer pair consisting of a combination of a modified primer consisting of the complementary sequence of the modified base sequence of SEQ ID NO: 6 and a modified primer consisting of the complementary sequence of the modified base sequence of SEQ ID NO: 10.
(c8-1) A primer pair consisting of a combination of a primer consisting of the complementary sequence of SEQ ID NO: 6 and a primer consisting of the complementary sequence of SEQ ID NO: 12.
(c8-2) A primer pair consisting of a combination of a modified primer consisting of the complementary sequence of the modified base sequence of SEQ ID NO: 6 and a primer consisting of the complementary sequence of SEQ ID NO: 12.
(c8-3) A primer pair comprising a combination of a primer consisting of the complementary sequence of SEQ ID NO: 6 and a primer consisting of the complementary sequence of the modified base sequence of SEQ ID NO: 12.
(c8-4) A primer pair consisting of a combination of a modified primer consisting of the complementary sequence of the modified base sequence of SEQ ID NO: 6 and a modified primer consisting of the complementary sequence of the modified base sequence of SEQ ID NO: 12.

Among these primer pairs, primer pair (a7) and primer pair (b7-1)∼(b7-3), (c7-1)~(c7-4) are preferable.
The primer pairs included in the groups (a) to (c) above have a common function in that they specifically hybridize within the structural gene possessed by mecA gene and amplify DNA fragments specific to mecA gene.

It is noted that the primer pairs of the present invention are found after the presence or absence of an effect is confirmed based on combinations of a considerable amount of various primers, and cannot be simply a level of ratification of the effect. It is also noted that as a result of the sequence search, it is confirmed that the primer pairs for detecting mecA genes are novel combinations.
The present invention does not prevent the combination of a plurality of known primers for bacteria, fungi, and viruses other than the primer pair of the present invention.
Primer concentrations in the PCR are not particularly limited, but generally, the PCR concentration ranges from 0.05pM to 1.0µM, and the PCR concentration is appropriately set according to consideration.

### <PCR enzyme >

The PCR enzyme used in the present invention is preferably a thermostable DNA polymerase derived from a thermostable organism, and more preferably from prokaryotes such as methanogen, thermophilic eosinophiles, thermophiles, and hyperthermophiles.
Specific examples of thermostable DNA polymerases for use herein include thermostable DNA polymerases derived from Thermus aquaticus (Thermus aquaticus), Thermus thermophilus, Bacillus stearothermophilus, Thermococcus gorgonarius, Thermococcus kodakaraensis KOD1, Pyrococcus woesei, Pyrococcus furiosus, Aeropyrum pernix, Aquifex aeolicus, Sulfolobus tokodaii, Pyrolobus fumarii, or Methanopyrus kandleri.
The thermostable DNA polymerase may be a thermostable DNA polymerase artificially synthesized by genetic engineering.

### <Other reagents>

Reagents other than primer pairs used for PCR analysis in the present invention can be used in known combinations. Reagents required for the measurements include enzymes for PCR, pH-buffer solution, dNTP, Mg²⁺ source, sterile water, and the like. In the real-time PCR analysis, a labeling substance such as a fluorescent dye is necessary in addition to the above.

The pH buffer is used to adjust the pH to 7.0 to 10.0, more preferably pH8 0 to 9.0, as a sample for PCR-analysis. Specific examples include Tris hydrochloric acid buffer. For example, Tris hydrochloride buffer is used at 5mM to 100mM.dNTP is a source of nucleosides for PCR-based DNA-amplification and requires four dATP, dCTP, dGTP, dTTP types. dNTP may be chemically modified for use in the hot start method, for example, CleanAmp™dNTP made by TriLink BioTechnologies, Inc. The amounts used are dATP, dCTP, dGTP, dTTP at concentrations of about 0.1 mM to 0.2mM, respectively.

Mg²⁺ is required for PCR-based DNA-amplification. Examples of Mg²⁺ source include MgCl₂, MgSO₄. Preferred is MgCl₂, which is used in amounts ranging from 0.5mM to 5.0mM as appropriate depending on the study. Fluorescent dyes are used for the detection of DNA amplification products by real-time PCR, as well as for the measurement of Tm values of amplified DNA fragments, including, for example, methods using intercalators with labeling functions. Examples of intercalators include ethidium bromide, SYBR Green I and Resolight (Roche, Inc), and EvaGreen (Biotium, Inc) and BOXTO (tataa biocentre). Preferable intercalators are EvaGreen and Resolight. The amount used is in accordance with the manufacturer's recommendations for the fluorescent dyes used.
Other kits may include bacterial genomic DNA for use as a positive control or sterile water for use as a negative control for PCR.

As a container for PCR analysis measurement (for example, an analysis measurement tube) used in the present invention, a container having a shape or a structure corresponding to a measurement instrument can be selected and used. If the one recommended by the source of the measurement instrument is used, it can be used for the measurement without any particular problem.
Other necessary instruments for practicing the present invention include tools widely used in molecular biology experiments, such as pipettes, pipette tips, 1.5ml microtubes, and devices widely used in molecular biology experiments, such as PCR, clean benches, tube centrifuges, and the like.

### <Kit embodiment>

A mecA gene detection kit of the present invention is a kit for detecting a mecA gene by analyzing DNA fragments amplified by the primer pairs of the present invention. Analysis of DNA fragments as amplification products can be performed by known methods. For example, analysis of DNA fragments as amplification products can be performed by molecular weight analysis, Tm value measurement (melting curve analysis), or the like. Two or more types of analysis methods may be combined. Preferably, the measurement of the Tm value, for which purpose real-time PCR is suitably used. The molecular weight can be analyzed by electrophoresis, mass spectrometry, or the like. A mecA gene detection kit according to the present invention comprises one or more primer pairs of the groups (a) to (c) described above. In addition to the primers, it may optionally contain at least one reagent and/or component selected from reagents such as PCR enzymes, pH-buffers, MgCl₂, dNTP (or CleanAmp™dNTP) and sterile water, components such as PCR assay containers (e.g., analysis measurement tubes) and other required instruments. Furthermore, in the analysis by real-time PCR, a fluorescent dye can be added as a component. Depending on various embodiments of the PCR method, components necessary for the PCR method used in addition to these components may be added to the kit. Each of these reagents or components may be packaged in any form, such as individually, partially, or all in one piece. Specifically, for example, the following mode is adopted.
1) Each reagent to be used is divided.
2) Three components, i.e., pre-mixed mixture, one primer pair and PCR enzyme are separately packed, and the pre-mixed mixture includes pH buffers, MgCl₂, dNTP (or CleanAmp™dNTP) (and the fluorescent dye in real-time PCR analysis).
3) Two components, i.e., pre-mixed mixture and PCR enzyme are separately packed, and the pre-mixed mixture includes pH buffer, MgCl₂, dNTP (or CleanAmp™dNTP) (and the fluorescent dye in real-time PCR analysis), one primer pair.
4) One component, i.e., pre-mixed mixture is packed, and the pre-mixed mixture includes pH buffers, MgCl₂, dNTP (or CleanAmp™dNTP) (and the fluorescent dye in real-time PCR analysis), one primer pair, and PCR enzyme.

When the kit has a plurality of primer pairs, the primer pairs are separately packaged. When a plurality of mixtures containing different primer pairs are used in the construction of the kit, each mixture is packaged separately.

### <Application fields/samples>

The inventive mecA gene detection kits can be used for mecA gene detection in various fields such as medical, food, environmental analyses, etc. In addition, it is used in conjunction with a rapid identification method of the infection causing bacteria in Patent Document 1, it can be used to simultaneously and rapidly perform the causing bacteria identification and mecA gene detection.
The test sample is not particularly limited and can be applied to a wide range of specimens. Specifically, in the medical field, blood, cerebrospinal fluid, lacrimal fluid, aqueous humor, amniotic fluid, joint fluid, saliva, nasal wipe, urine, other body fluids, adhering substances of medical devices such as catheters, and the like can be cited. In the food field, there are food itself, liquid in the process of production, or solid feed, equipment deposits in the manufacturing process, and the like.

### <DNA extraction>

When mecA gene detection is performed in a sample using the inventive mecA gene detection kits, DNA needs to be extracted in advance from bacteria that may be present in the sample. As DNA extraction method, an alkali dissolution method, a boiling method, a phenol extraction method, a bead crushing method, and the like are known at present, and various DNA extraction kits are also sold from manufacturers.
The DNA extraction method in the present invention is not particularly limited, but since the optimal method differs depending on the sample, it is desirable to select a method corresponding to the target sample.

### <PCR analysis>

The primer pairs of the present invention are used in PCR methods. As the PCR method, various PCR methods can be used as long as they are PCR methods for amplifying a target gene for detecting a mecA gene. A preferred analytical method is real-time PCR, more preferably a combination of real-time PCR and melting curve analysis for Tm value measurement. In this case, the real-time PCR apparatus used is not particularly limited. Rotor Gene Q manufactured by Qiagen Corporation used in the embodiment of the present application is an example of a real-time PCR apparatus which can be suitably used.
In PCR and real-time PCR, commonly known apparatuses, techniques, and the like may be used.
The conditions of the temperature cycle in PCR (temperature, time, temperature rise/fall rate, and number of cycles) are not particularly limited, and may be appropriately set according to the properties of the primer used, the enzyme for PCR, the template, and the like, and the sensitivity of the DNA detection method after PCR. Much literature is already known on the setting of these conditions.
In PCR, the steps of thermal denaturing the template double-stranded DNA, annealing the primers, and extending the DNA with an enzyme for PCR are generally repeated. The step of thermal denaturation may be a temperature and a time at which the template double-stranded DNA is dissociated into single strands, and is set, for example, at 90° C to 98° C for several seconds to several minutes. At the start of PCR, a thermal denaturation process of several minutes to 10 minutes is often added only to the first cycle. The annealing step of the primer is set in accordance with the base sequence and the number of bases of the primer, but it is often set at 40° C to 72° C for several seconds to several tens of seconds. In the DNA extending step, the temperature is depending on the properties such as the optimum temperature of the enzyme for PCR for example, 58° C to 76° C is generally used, and the required time is estimated and set based on the strand length of the DNA to be amplified and the DNA synthesis rate of the enzyme for PCR. The target DNA is amplified by repeating the steps of thermal denaturation, annealing, and extension, and the number of repetitions may be appropriately changed depending on the amount of template DNA, the amount of enzyme for PCR, and the sensitivity of the DNA detection method after PCR, but 10 to 50 times are exemplified as general examples. When the annealing temperature and the DNA extension temperature are approximately the same, both steps can be performed simultaneously.
Also in real-time PCR, conditions for thermal denaturation, annealing, and DNA extension necessary for DNA amplification, and the number of repetitions of these steps are the same as described above for PCR. In real-time PCR, it is possible to quantify or estimate the amount of amplified DNA by measuring the fluorescence intensity derived from the intercalator before and after the step of DNA extension. The temperature at which the fluorescence intensity is measured may be maintained at the temperature of DNA extension, for example in the implementation using intercalator. The temperature may be set between the Tm value of the target DNA and the Tm value of the non-specifically amplified non-target DNA (also referred to as non-specifically amplified DNA) such as intermediate temperature, by utilizing the fact that the Tm value of the non-specifically amplified DNA is relatively low when the strand length of the target DNA to be amplified is relatively long and the Tm value thereof is relatively high. In this way, in particular, in the implementation using intercalator, only the non-specifically amplified DNA such as primer dimer is dissociated from the double strand to the single strand, and the amount of DNA quantitated or estimated from the fluorescence intensity can be regarded as the target DNA.
Further, in the real-time PCR, after completion of the DNA amplification step, the Tm value of the amplified DNA can be measured by melting curve analysis. In melting curve analysis, dissociation of DNA from double strand to single strand in response to temperature change is observed, but the temperature and detection conditions are not particularly limited. Generally, by proceeding through the steps of thermal denaturation (Denaturation) (90° C to 98° C), duplex formation (Annealing) (40° C to 80° C), and Melting (gradually increasing from the temperature of duplex formation to around 98° C), the change in fluorescent intensity at the step of melting can be monitored to obtain melting curves from which Tm values can be obtained. Such measurements are possible in many models of real-time PCR devices and can be performed according to how the instrument is used.

### <Detection method>

As a detection method of a mecA gene in a sample, the method having the following steps can be used.
(1) A step of performing PCR using genomic DNA of the bacterium prepared from the sample, primer pairs for obtaining an amplified product containing a mecA gene, and PCR reagents such as thermostable DNA polymerases.
(2) A step of detecting a mecA gene in the amplification product obtained by the PCR step or detecting a mecA gene in the sample by analyzing the amplification product.
When a plurality of primer pairs are used, the PCR step (1) is performed separately for each of the plurality of primer pairs. That is, one primer pair is added to one reaction solution for PCR, and a plurality of reaction solutions to which different primer pairs are added are individually used to perform the PCR step (1).
By using at least one of the present primer pairs as a primer pair for obtaining an amplified product containing mecA gene, a highly sensitive methicillin-resistance gene (mecA) can be detected.
In this detecting method, it is preferable that the amplifying step is performed under suppression of amplification of genes other than mecA gene as the target gene (non-target genes). Hot start PCR can be used to suppress the amplification of the non-target genes. An example is a hot start method using an anti-DNA polymerase antibody. In this case, it is preferable to use excessive amounts of anti-DNA polymerase antibody against 1U of the thermostable DNA polymerase. Further, as disclosed in Patent Document 1 and International Publication No. WO 2010/082640, a hot start method by reversibly chemically modifying a DNA polymerase can also be suitably used. Furthermore, a hot start method using a chemically modified dNTP or using primers chemically modified as described in US2007/0281308A1 can also be suitably used. In addition, a hot start method by physically isolating a DNA polymerase and components (e.g., primers, dNTP, or Mg²⁺ salts) essential for amplifying DNA by the DNA polymerase by using waxes or the like which are melted by heat can be suitably used.

The detection of the target gene in the amplification product in the step of detecting the amplification product can be carried out by measuring the Tm value by real-time PCR using an intercalator having a fluorescent label for detection. Preferred intercalators are Cyber Green I, EvaGreen, Resolight and BOXTO, more preferably EvaGreen and Resolight.
In the detection step by Tm value measurement using real-time PCR, the amplification product of the target gene can be detected, and the amplification product of the other gene other than the target gene can be performed as non-detection. As a method for this purpose, it is preferable to set a condition in which the amplification product of the target gene can be detected and the amplification product of the other gene other than the target gene is not detected by
(1) selecting a primer in which the melting temperature (TmA) of the target gene amplification product is higher than the melting temperature (TmB) of the amplification product of the non-target gene, and
(2) detecting the amplification product at a temperature between TmA and TmB.
Further, the amplification step and the detection step can be performed by real-time PCR using a display device for displaying the amount of the amplification product, and a method in which the amplification product of the non-target gene is not displayed on the display device can be used. Analysis of the amplification product in the detection step can be performed by analysis of the amplification product which develops and visualizes the amplification product by electrophoresis on a gel or the like. Analysis of the amplification product can also be performed by analysis of the amplification product by decoding the base sequence of the amplification product. Furthermore, the amplification product can be analyzed by a method in which the molecular weight of the amplification product is measured and analyzed by a mass spectrometer.

### EXAMPLES:

### (Example 1) Primary Screening

The genome DNA extracted from the culture medium of MRSA (RIKEN CORPORATION, JCM31453) using High pure PCR template preparation kit (Roche) was used as template to amplify the entire length of mecA ORF by performing PCR using the primers of SEQ ID NO: 15 and SEQ ID NO: 16 with the reaction solution compositions shown in Table 1. Next, dilute series of mecA amplification products were prepared, and PCR was performed using a pair of comparative primers consisting of a forward primer consisting of SEQ ID NO: 13 and a reverse primer consisting of SEQ ID NO: 14 with the reaction solution composition shown in Table 1, and the concentration of the amplification product of mecA where the number of rising cycles was approximately 20 was determined, and used as template. Methicillin-resistant Staphylococcus aureus, JCM31453 is publicly available from Microbe Division of RIKEN BioResource Research Center (RIKEN BRC-JCM, 3-1-1, Takanodai, Tsukuba-shi, Ibaraki 305-0074, Japan).
Next, in order to evaluate the prepared primers, PCR was performed on 313 primer pairs with the reaction solution composition shown in Table 1. A rotor-gene Q MDx 5plex HRM(QIAGEN) was used as the real-time PCR device. The PCR reaction conditions were repeated 40 times at 94° C for 10 seconds, 65° C for 10 seconds, and 72° C for 30 seconds, after heating at 95° C for 5 minutes. 88 pairs were selected which showed an improvement in the number of rise cycles, the shape of the peak in High resolution melting analysis (HRM), the intensities of the bands in the electrophoresis, and the presence or absence of non-specific products in the electrophoresis compared with the results of the primer pairs for comparison.

### (Example 2) Secondary Screening

The genome DNA extracted from the culture medium of MRSA (RIKEN CORPORATION, JCM31453) using High pure PCR template preparation kit (Roche) was used as template with a solution adjusted so that the number of rise cycles becomes about 20 cycles with 50µg/ml human genomic DNA solution (invirogen).
88 primer pairs were evaluated by performing PCR with the reaction solution composition shown in Table 1. A rotor-gene Q MDx 5plex HRM(QIAGEN) was used as the real-time PCR device. The PCR reaction conditions were repeated 40 times at 94° C for 10 seconds, 65° C for 10 seconds, and 72° C for 30 seconds, after heating at 95° C for 5 minutes. 57 pairs were selected which showed an improvement in the number of rise cycles, the shape of the peak in HRM, the intensity of the band in electrophoresis, and the presence or absence of non-specific products in electrophoresis compared with the results of the primer pairs for comparison.

### (Example 3) Third Screening

The genome DNA extracted from the culture medium of MRSA (RIKEN CORPORATION, JCM31453) using High pure PCR template preparation kit (Roche) was used as template with a solution adjusted so that the concentration becomes 10.4 molecule/reaction solution with 50 pg/reaction ml human genomic DNA solution (invitrogen). 57 primer pairs were evaluated by performing PCR in triplicate measurements using the reaction solution composition shown in Table 1. A rotor-gene Q MDx 5plex HRM(QIAGEN) was used as the real-time PCR device. The PCR reaction conditions were repeated 40 times at 94° C for 10 seconds, 65° C for 10 seconds, and 72° C for 30 seconds, after heating at 95° C for 5 minutes. 9 pairs were selected which showed an improvement in amplifications in all triplicate measurements, Height of dF/dT in HRM (channel A), presence or absence of primer dimers, and presence or absence of peaks of non-specific products compared to results with comparative primer pairs.

### (Example 4) Final screening

The genome DNA extracted from the culture medium of MRSA (RIKEN CORPORATION, JCM31453) using High pure PCR template preparation kit (Roche) was used as template with a solution adjusted so that each concentration becomes concentrations (copy number/reaction solution: indicated as Copies/Reaction in Table 2) with 50 pg/reaction ml human genomic DNA solution (invitrogen). 9 primer pairs were evaluated by performing PCR in triplicate measurements using the reaction solution composition shown in Table 1. A rotor-gene Q MDx 5plex HRM(QIAGEN) was used as the real-time PCR device. The PCR reaction conditions were repeated 40 times at 94° C for 10 seconds, 65° C for 10 seconds, and 72° C for 30 seconds, after heating at 95° C for 5 minutes. The detection limit was the lowest concentration at which amplification was observed in all triplicate measurements. Whether or not the target amplification product was obtained was confirmed by measurement of the Tm value.
As a result, 8 pairs were selected which showed an improvement in the detection limit compared with the case where detection was performed using the comparative primer pair shown in Patent Document 1, which is the prior application. The results are given in Table 2. In addition, SEQ ID NOs: 13 and 14 of the primer pair for comparison and the base sequences of the 8 pairs selected are shown in Table 3.

### (Example 5) Comparative of Methicillin Resistant Strains and Methicillin Sensitive Strains

A genome DNA solution extracted from the culture mediums of Staphylococcus aureus (methicillin-sensitive Riken subdivision strain, JCM2151) and MRSA (methicillin-resistant Riken subdivision strain, JCM16555) using High pure PCR template preparation kit (Roche) was used as template. The primer pair of SEQ ID NO: 6 and SEQ ID NO: 10 were evaluated by performing PCR with the reaction solution composition shown in Table 1. A rotor-gene Q MDx 5plex HRM(QIAGEN) was used as the real-time PCR device. The PCR reaction conditions were repeated 40 times at 94° C for 10 seconds, 65° C for 10 seconds, and 72° C for 30 seconds, after heating at 95° C for 5 minutes. Whether or not the target amplification product was obtained was confirmed by the presence or absence of a peak in the vicinity of 81° C by measuring the Tm value. The results of methicillin-resistant strain are shown in FIG. 1(a) and the results of methicillin-sensitive strain are shown in FIG. 1(b).

**Table 1**

| Reaction solution composition | | |
|---|---|---|
| Reagent name | Volume | |
| Template | 2.0 | µL |
| 10X Thnuder Taq Buffer | 2.0 | µL |
| 25mM MgCl2 | 1.6 | µL |
| e-DNAP | 1.0 | Unit |
| 2mM CleanAmp-dNTP | 2.0 | µL |
| EvaGreen (Biotium) | 1.0 | µL |
| 10mM forward primer | 0.6 | µL |
| 10mM reverse primer | 0.6 | µL |
| DW | Adequate dose | |
| Total | 20.0 | µL |

| | | |
|---|---|---|
| (DW : Deionized water) | | |

**Table 2**

| Primer Pair | Forward primer | Reverse primer | detection limit (Copies/Reaction) |
|---|---|---|---|
| a1 | SEQ ID NO: 3 | SEQ ID NO: 7 | 5.2 |
| a2 | SEQ ID NO: 2 | SEQ ID NO: 9 | 5.2 |
| a3 | SEQ ID NO: 1 | SEQ ID NO: 8 | 2.6 |
| a4 | SEQ ID NO: 1 | SEQ ID NO: 9 | 10.4 |
| a5 | SEQ ID NO: 4 | SEQ ID NO:11 | 5.2 |
| a6 | SEQ ID NO: 5 | SEQ ID NO:12 | 5.2 |
| a7 | SEQ ID NO: 6 | SEQ ID NO:10 | 2.6 |
| a8 | SEQ ID NO: 6 | SEQ ID NO:12 | 2.6 |
| Comparison | SEQ ID NO:13 | SEQ ID NO:14 | 100.4 |

**Table 3**

| | sequence (5'→3') |
|---|---|
| SEQ ID NO: 1 | CTGCTATCCACCCTCAAACAG |
| SEQ ID NO: 2 | ACTGCTATCCACCCTCAAAC |
| SEQ ID NO: 3 | GAAGATGGCTATCGTGTCAC |
| SEQ ID NO: 4 | ATCTTGGGGTGGTTACAACG |
| SEQ ID NO: 5 | TAGCACTCGAATTAGGCAGTAAG |
| SEQ ID NO: 6 | AGCTGATTCAGGTTAGGGAC |
| SEQ ID NO: 7 | AATCTGGAACTTGTTGAGCAG |
| SEQ ID NO: 8 | AACGTTGTAACCACCCCAAG |
| SEQ ID NO: 9 | TGTAACGTTGTAACCACCCC |
| SEQ ID NO: 10 | GATTTTGGCATTGTAGCTAGCC |
| SEQ ID NO: 11 | ACCACCCAATTTGTCTGCC |
| SEQ ID NO: 12 | GTACCGGATTTGCCAATTAAG |
| SEQ ID NO: 13 | CAAACTACGGTAACATTGATCGC |
| SEQ ID NO: 14 | ATGTATGCTTTGGTCTTTCTGC |
| SEQ ID NO: 15 | ATGAAAAAGATAAAAATTGTTC |
| SEQ ID NO: 16 | TTATTCATCTATATCGTAT |

### Industrial Applicability

From the test results described in Table 2, it was found that the detection method of the present invention using the primer pairs of the present invention is capable of detecting extremely small amounts of mecA gene such as 10 copies or less in one reaction. The present detection method is sufficiently practical and has wide applicability because the detection method can detect extremely rare mecA gene in an extremely small amount of an analyte with high sensitivity, high accuracy, and quickly. The present primer pairs, detection methods, and detection kits can be used to detect trace amounts of mecA gene in a variety of analytes that occur in the medical field, the food field, and environmental analyses.

## Claims

1. A primer pair for detecting a mecA gene (methicillin-resistance gene), wherein the primer pair is one primer pair selected from the group consisting of (a) to (c) below.
(a) A primer pair comprising a combination of SEQ ID NO:3 and SEQ ID NO:7, a combination of SEQ ID NO:2 and SEQ ID NO:9, a combination of SEQ ID NO:1 and SEQ ID NO:8, a combination of SEQ ID NO:1 and SEQ ID NO:9, a combination of SEQ ID NO:4 and SEQ ID NO:11, a combination of SEQ ID NO:5 and SEQ ID NO: 12, a combination of SEQ ID NO:6 and SEQ ID NO:10 or a combination of SEQ ID NO:6 and SEQ ID NO: 12,
(b) In (a) above, a primer pair in which one or two bases are added, deleted or substituted to the extent that a part of the base sequence of one or both primers does not impair the function as a primer,
(c) A primer pair comprising a complementary sequence corresponding to the base sequence of (a) or (b) above.

2. The primer pair according to claim 1, wherein the primer pair is a primer pair consisting of a combination of SEQ ID NO: 6 and SEQ ID NO: 10, or a primer pair in which one or two bases are added, deleted, or substituted to the extent that a part of the base sequence of one or both primers of the combination does not impair the function as a primer.

3. A mecA gene detection kit comprising a primer pair, wherein said primer pair is at least one primer pair selected from the group consisting of (a) to (c) below.
(a) A primer pair comprising a combination of SEQ ID NO:3 and SEQ ID NO:7, a combination of SEQ ID NO:2 and SEQ ID NO:9, a combination of SEQ ID NO:1 and SEQ ID NO:8, a combination of SEQ ID NO:1 and SEQ ID NO:9, a combination of SEQ ID NO:4 and SEQ ID NO:11, a combination of SEQ ID NO:5 and SEQ ID NO:12, a combination of SEQ ID NO:6 and SEQ ID NO:10 or a combination of SEQ ID NO:6 and SEQ ID NO:12,
(b) In (a) above, a primer pair in which one or two bases are added, deleted or substituted to the extent that a part of the base sequence of one or both primers does not impair the function as a primer,
(c) A primer pair comprising a complementary sequence corresponding to the base sequence of (a) or (b) above.

4. The kit according to claim 3, wherein the primer pair is a primer pair consisting of a combination of SEQ ID NO: 6 and SEQ ID NO: 10, or a primer pair in which one or two bases are added, deleted, or substituted to the extent that a part of the base sequence of one or both primers of the combination does not impair the function as a primer.

5. The kit according to claim 3 or 4 comprising at least one of an enzyme for PCR, a reagent for PCR and an instrument for PCR.

6. A detection method of a mecA in a sample, comprising:
a PCR step of performing PCR using DNA prepared from the sample and a primer pair for amplifying the DNA; and
a step of detecting a mecA gene in the sample by detecting a mecA gene in an amplification product obtained by the PCR step or by analyzing the amplification product,
wherein the primer pair used in the PCR step is at least one primer pair selected from the group consisting of (a) to (c) below.
(a) A primer pair comprising a combination of SEQ ID NO:3 and SEQ ID NO:7, a combination of SEQ ID NO:2 and SEQ ID NO:9, a combination of SEQ ID NO:1 and SEQ ID NO:8, a combination of SEQ ID NO:1 and SEQ ID NO:9, a combination of SEQ ID NO:4 and SEQ ID NO:11, a combination of SEQ ID NO:5 and SEQ ID NO: 12, a combination of SEQ ID NO:6 and SEQ ID NO:10 or a combination of SEQ ID NO:6 and SEQ ID NO: 12,
(b) In (a) above, a primer pair in which one or two bases are added, deleted or substituted to the extent that a part of the base sequence of one or both primers does not impair the function as a primer,
(c) A primer pair comprising a complementary sequence corresponding to the base sequence of (a) or (b) above.

7. The detection method according to claim 6, wherein the primer pair is a primer pair consisting of a combination of SEQ ID NO: 6 and SEQ ID NO: 10, or a primer pair in which one or two bases are added, deleted, or substituted to the extent that a part of the base sequence of one or both primers of the combination does not impair the function as a primer.


SEQUENCE LISTING
<110>  Mitsui chemicals, inc.
<120>  A primer pair to amplify mecA gene, a kit for detection of mecA gene
and a method of detection of mecA gene
<130>  18-0075-MCI
<150>  JP 2017-198576
<151>  2017-10-12

<160>  16

<210>  1
<211>  21
<212>  DNA
<213>  Artificial

<220>
<223>  Oligonucleotide to act as a primer

<400>  1
ctgctatcca ccctcaaaca g                    21


<210>  2
<211>  20
<212>  DNA
<213>  Artificial

<220>
<223>  Oligonucleotide to act as a primer

<400>  2
actgctatcc accctcaaac                      20


<210>  3
<211>  20
<212>  DNA
<213>  Artificial

<220>
<223>  Oligonucleotide to act as a primer

<400>  3
gaagatggct atcgtgtcac                      20


<210>  4
<211>  20
<212>  DNA
<213>  Artificial

<220>
<223>  Oligonucleotide to act as a primer

<400>  4
atcttggggt ggttacaacg                      20


<210>  5
<211>  23
<212>  DNA
<213>  Artificial

<220>
<223>  Oligonucleotide to act as a primer

<400>  5
tagcactcga attaggcagt aag                                          23


<210>  6
<211>  20
<212>  DNA
<213>  Artificial

<220>
<223>  Oligonucleotide to act as a primer

<400>  6
agctgattca ggttacggac                                              20


<210>  7
<211>  21
<212>  DNA
<213>  Artificial

<220>
<223>  Oligonucleotide to act as a primer

<400>  7
aatctggaac ttgttgagca g                                            21


<210>  8
<211>  20
<212>  DNA
<213>  Artificial

<220>
<223>  Oligonucleotide to act as a primer

<400>  8
aacgttgtaa ccaccccaag                                              20


<210>  9
<211>  20
<212>  DNA
<213>  Artificial

<220>
<223>  Oligonucleotide to act as a primer

<400>  9
tgtaacgttg taaccacccc                                              20


<210>  10
<211>  22
<212>  DNA
<213>  Artificial

<220>
<223>  Oligonucleotide to act as a primer

<400>  10
gattttggca ttgtagctag cc                                              22


<210>  11
<211>  19
<212>  DNA
<213>  Artificial

<220>
<223>  Oligonucleotide to act as a primer

<400>  11
accacccaat ttgtctgcc                                                  19


<210>  12
<211>  21
<212>  DNA
<213>  Artificial

<220>
<223>  Oligonucleotide to act as a primer

<400>  12
gtaccggatt tgccaattaa g                                               21


<210>  13
<211>  23
<212>  DNA
<213>  Artificial

<220>
<223>  Oligonucleotide to act as a primer

<400>  13
caaactacgg taacattgat cgc                                             23


<210>  14
<211>  22
<212>  DNA
<213>  Artificial

<220>
<223>  Oligonucleotide to act as a primer

<400>  14
atgtatgctt tggtctttct gc                                              22

<210>  15
<211>  22
<212>  DNA
<213>  Artificial

<220>
<223>  Oligonucleotide to act as a primer

<400>  15
atgaaaaaga taaaaattgt tc                                              22

<210>  16

<211>  19
<212>  DNA
<213>  Artificial

<220>
<223>  Oligonucleotide to act as a primer

<400>  16
ttattcatct atatcgtat                    19